# EUROPEAN PATENT APPLICATION

(11) **EP 2 910 192 A1**
(43) Date of publication of application: **26.08.2015**
(21) Application number: 15155730.3
(22) Date of filing: 19.02.2015
(51) Int. Cl.: A61B 8/08, A61B 8/00

(54) **Ultrasonic measurement apparatus and ultrasonic measurement method**

(30) Priority: 21.02.2014 JP 2014031418
(71) Applicant: Seiko Epson Corporation, Tokyo 163 (JP)
(72) Inventor: Hyuga, Takashi, Nagano, 392-8502 (JP)
(74) Representative: Hoffmann Eitle

(57) **Abstract**

Ultrasound measurement data is acquired from a blood vessel (3) through the surface of the body, the blood vessel having a branch point (4). The branch point is detected from A-mode data. Then, the distance that the ultrasonic probe (16) has moved on the surface of the body along the longitudinal direction of the blood vessel is determined from the B-mode image, and the user is notified when a predetermined position has been reached for performing a vascular function measurement (e.g. measurement of intima media thickness of a carotid artery). It is thus possible to realise accurate positioning of the probe at the same position on the blood vessel, e.g. for periodic measurements.

## Description

### BACKGROUND

### 1. Technical Field

The present invention relates to an ultrasonic measurement apparatus that measures biological information using an ultrasonic wave.

### 2. Related Art

As an example of measuring biological information with an ultrasonic measurement apparatus, the evaluation of a vascular function or the determination of a vascular disease is performed.

For example, the intima media thickness (IMT) of the carotid artery, which is an indicator of arteriosclerosis, is measured. In the measurement relevant to the IMT or the like, it is necessary to locate the carotid artery and appropriately determine the measurement point. Typically, the operator places an ultrasonic probe on the neck, locates the carotid artery to be measured while watching a B-mode image displayed on the monitor, and manually sets the found carotid artery as a measurement point.

Although skill is required in order to execute such a series of measurement operations quickly and locate the carotid artery appropriately in the related art, a function to assist the measurement operation has been devised in recent years. For example, JP-A-2008-173177 discloses a method of detecting the vessel wall automatically using the strength of a reflected wave signal from the body tissue, which is obtained by processing the amplitude information of the received reflected wave, and the moving speed of the body tissue, which is obtained by processing the phase information of the received reflected wave.

Incidentally, when periodically measuring the biological information, it is desirable to perform measurement at the same position on the blood vessel from the point of view of measurement conditions error. However, in such periodic measurement performed every few days or every few weeks, an ultrasonic probe is not continuously fixed to the subject. Accordingly, it has been difficult to perform measurement at substantially the same position on the blood vessel.

### SUMMARY

An advantage of some aspects of the invention is to easily realize steady and accurate positioning for the blood vessel to be subjected to ultrasonic measurement.

A first aspect of the invention is directed to an ultrasonic measurement apparatus including: an ultrasonic wave transmission and reception unit that transmits and receives an ultrasonic wave to and from a blood vessel through a surface of a body, the blood vessel having a part satisfying predetermined tissue conditions; an acquisition unit that acquires measurement data, which is obtained by measuring a cross section of the body with an ultrasonic wave, based on a received signal of the ultrasonic wave transmission and reception unit; a detection unit that detects the part based on the measurement data; and a movement detection unit that detects that the ultrasonic wave transmission and reception unit has moved on the surface of the body along a longitudinal direction of the blood vessel and has reached a predetermined position for performing predetermined vascular function measurement, from the detection of the detection unit, based on the measurement data.

According to the first aspect of the invention, when an ultrasonic probe is placed on the blood vessel of the subj ect and is moved in the longitudinal direction of the blood vessel, it is automatically detected that the ultrasonic probe has arrived at the predetermined position for performing vascular function measurement. That is, just by moving the ultrasonic probe along the blood vessel by the operator, it is possible to perform steady and accurate positioning for the blood vessel to be subjected to ultrasonic measurement.

A second aspect of the invention is directed to the ultrasonic measurement apparatus according to the first aspect of the invention, wherein, assuming that the tissue conditions are that the blood vessel branches into a plurality of branch blood vessels, the detection unit detects a branch point at which the blood vessel branches into the plurality of branch blood vessels.

According to the second aspect of the invention, since the tissue conditions can be a branch point that is easily detected by the ultrasonic wave, it is possible to improve the positioning accuracy.

A third aspect of the invention is directed to the ultrasonic measurement apparatus according to the first or second aspect of the invention, wherein the movement detection unit calculates a moving distance by comparing the measurement data in time series, and detects an arrival at the predetermined position when the moving distance from detection of the detection unit has amounted to a predetermined distance.

According to the third aspect of the invention, it is possible to calculate the moving distance using the measurement data based on the ultrasonic wave. Therefore, it is not necessary to provide a separate measurement unit for calculating the moving distance.

A fourth aspect of the invention is directed to the ultrasonic measurement apparatus according to any one of the first to third aspects of the invention, wherein the detection unit detects the part based on the measurement data relevant to a cross section of the blood vessel in a short-axis direction of the blood vessel, and the movement detection unit detects an arrival at the predetermined position based on the measurement data relevant to a cross section of the blood vessel in a long-axis direction of the blood vessel.

According to the fourth aspect of the invention, it becomes easy to detect the tissue conditions of the blood vessel by measuring the cross section of the blood vessel in the short-axis direction. In addition, it becomes easy to detect movement by measuring the cross section of the blood vessel in the long-axis direction. According to the fourth aspect of the invention, both can be used.

A fifth aspect of the invention is directed to the ultrasonic measurement apparatus according to any one of the first to fourth aspects of the invention, which further includes a notification unit that notifies that detection has been made by the movement detection unit.

According to the fifth aspect of the invention, it is possible to notify the operator of an arrival at the predetermined position.

When the invention is applied to carotid artery measurement, it is preferable that the ultrasonic measurement apparatus according to any one of the first to fifth aspects of the invention is configured, as a sixth aspect of the invention, such that the blood vessel is a carotid artery and the detection unit detects a branch point of an internal carotid artery and an external carotid artery.

A seventh aspect of the invention is directed to an ultrasonic measurement method including: acquiring measurement data, which is obtained by measuring a cross section of a body with an ultrasonic wave, based on a received signal of an ultrasonic wave transmission and reception unit for transmitting and receiving an ultrasonic wave to and from a blood vessel through a surface of the body, the blood vessel having a part satisfying predetermined tissue conditions; detecting the part based on the measurement data; and detecting that the ultrasonic wave transmission and reception unit has moved on the surface of the body along a longitudinal direction of the blood vessel and has reached a predetermined position for performing predetermined vascular function measurement, from the detection of the part, based on the measurement data.

According to the seventh aspect of the invention, it is possible to achieve the same effects as in the first aspect of the invention.

### BRIEF DESCRIPTION OF THE DRAWINGS

The invention will be described with reference to the accompanying drawings, wherein like numbers reference like elements.
Fig. 1 is a diagram showing an example of the system configuration of a biological information measuring apparatus.
Fig. 2 is a diagram for explaining the outline until a predetermined position for performing vascular function measurement (measurement target position) is detected.
Fig. 3 a diagram for explaining a method of detecting the branch point.
Fig. 4 is a diagram for explaining the calculation of the moving distance of the ultrasonic probe along the blood vessel from the detection timing of the branch point.
Fig. 5 is a diagram for explaining the principle of calculating the moving distance of the ultrasonic probe.
Fig. 6 is a block diagram showing an example of the functional configuration of an ultrasonic measurement apparatus.
Fig. 7 is a diagram showing an example of a program or data stored in a storage unit.
Fig. 8 is a flowchart for explaining the operation up to vascular function measurement.
Fig. 9 is a flowchart for explaining the operation up to vascular function measurement suitable for the configuration using an ultrasonic probe in which ultrasonic transducers are arrayed in one row.

### DESCRIPTION OF EXEMPLARY EMBODIMENTS

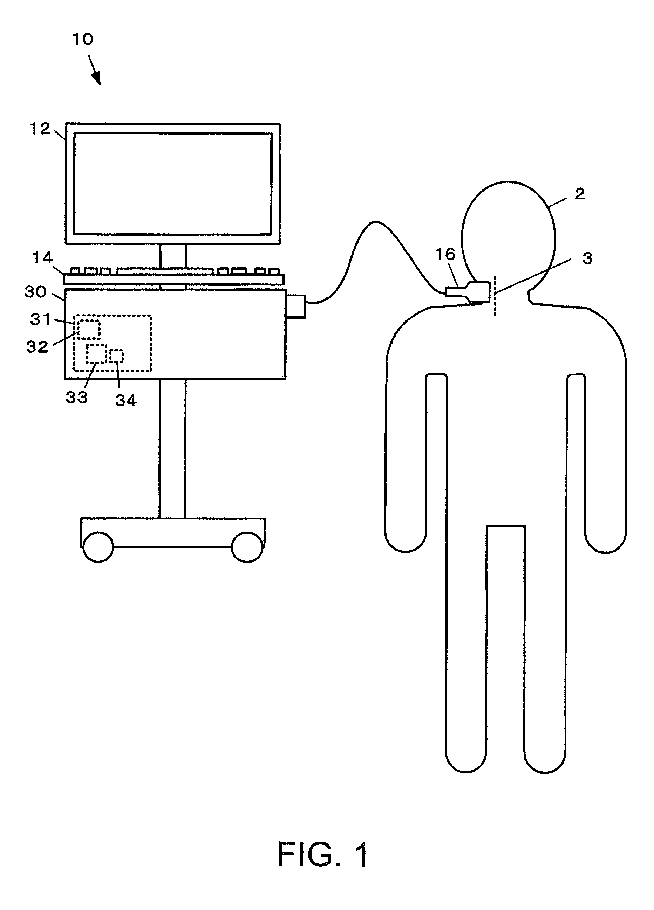

Fig. 1 is a diagram showing an example of the system configuration of an ultrasonic measurement apparatus 10 according to the present embodiment. The ultrasonic measurement apparatus 10 is an apparatus that measures biological information of a subject 2 by measuring the reflected waves of ultrasonic waves. In the present embodiment, vascular function information, such as the intima media thickness (IMT) of a carotid artery 3, is measured as a piece of biological information. Needless to say, it is also possible to measure other vascular function information, such as a blood vessel diameter or blood pressure estimated from the blood vessel diameter, in addition to the IMT.

The ultrasonic measurement apparatus 10 includes a touch panel 12 serving as a unit that displays a measurement result or operation information as an image and as an operation input unit, a keyboard 14 used for operation input, an ultrasonic probe 16, and a processing device 30. A control board 31 is mounted in the processing device 30, and is connected to each unit of the apparatus, such as the touch panel 12, the keyboard 14, and the ultrasonic probe 16, so that signal transmission and reception therebetween is possible.

Not only various integrated circuits, such as a central processing unit (CPU) 32 and an application specific integrated circuit (ASIC), but also a storage medium 33, such as an IC memory or a hard disk, and a communication IC 34 for realizing data communication with an external device are mounted on the control board 31. The processing device 30 realizes various functions according to the present embodiment, including ultrasonic measurement, by executing a control program stored in the storage medium 33 with the CPU 32 or the like.

Specifically, by the control of the processing device 30, the ultrasonic measurement apparatus 10 transmits an ultrasonic beam from the ultrasonic probe 16 to the tissue in the body of the subject 2 and receives the reflected wave. Then, by performing amplification and signal processing on a received signal of the reflected wave, it is possible to generate reflected wave data relevant to a structure in the body of the subject 2. Images of respective modes of a so-called A mode, B mode, M mode, and color Doppler are included in the reflected wave data. Measurement using an ultrasonic wave is repeatedly performed at predetermined periods. The measurement unit is referred to as a "frame".

By setting a region of interest (tracking point) in the reflected wave data as a reference, the ultrasonic measurement apparatus 10 can perform so-called "tracking" that tracks each region of interest between different frames and calculates the displacement.

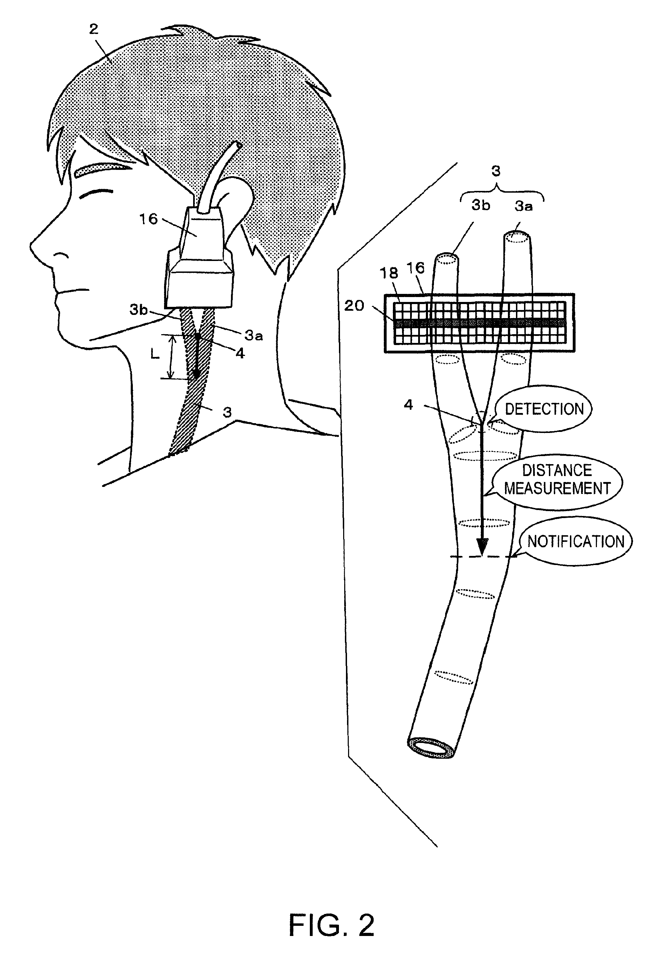

Fig. 2 is a diagram for explaining the outline until a predetermined position for performing vascular function measurement (hereinafter, referred to as a "measurement target position") is detected.

In the measurement, the subject 2 lies down on the bed with the left side of the neck (or the right side of the neck) upward.

The ultrasonic probe 16 of the present embodiment is of a type in which ultrasonic transducers are arrayed in a two-dimensional manner in the long side direction and the short side direction of an ultrasonic wave transmitting and receiving surface 18. The operator places the ultrasonic probe 16 at a position near the head in a relative posture in which the long side of the ultrasonic wave transmitting and receiving surface 18 traverses the short-axis direction of the carotid artery 3. Then, when the measurement is started, the operator moves the ultrasonic probe 16 slowly to the torso side along the carotid artery 3 while maintaining the posture.

When the operation for starting the measurement is detected, the ultrasonic measurement apparatus 10 starts ultrasonic measurement of the vascular short-axis cross section of the carotid artery 3 using ultrasonic transducers in a row in the long side direction (in the example shown in Fig. 2, a left and right direction of Fig. 2), among the ultrasonic transducers arrayed in a two-dimensional manner, and analyzes a received signal of the reflected wave for each measurement frame to detect a branch point 4 (vascular structural part satisfying predetermined tissue conditions) at which branching from the common carotid artery to an internal carotid artery 3a and an external carotid artery 3b occurs (detection process).

Then, when the branch point 4 is detected, a moving distance from the branch point 4 of the ultrasonic probe 16 is calculated by comparing measurement data relevant to the cross section of the carotid artery 3 in the long-axis direction in time series using ultrasonic transducers in a column in the short side direction (in the example shown in Fig. 2, an up and down direction of Fig. 2), among the ultrasonic transducers arrayed in a two-dimensional manner from the branch point 4, and it is detected that the moving distance has amounted to a predetermined distance L (distance measuring process). Then, the ultrasonic measurement apparatus 10 performs predetermined notification at a place where the moving distance has amounted to the predetermined distance L (notification process).

The operator becomes aware that the ultrasonic probe 16 has reached the measurement target position by the notification, and stops the movement of the ultrasonic probe 16. When an operation input for starting the measurement of predetermined vascular function information is detected (or when it is measured that a predetermined amount of time has passed from the notification information), the ultrasonic measurement apparatus 10 starts the measurement of the vascular function information with the ultrasonic probe 16.

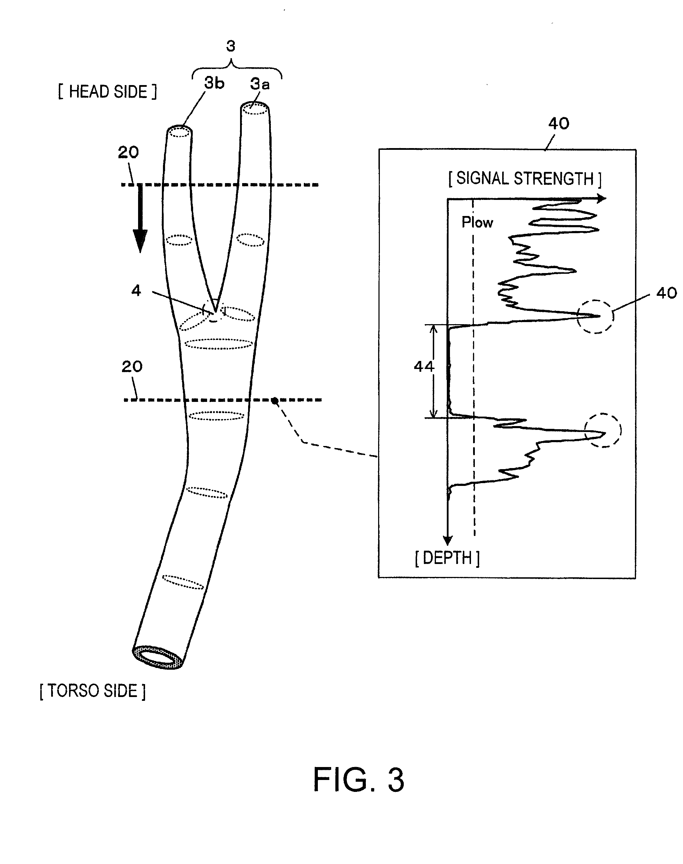

Fig. 3 is diagram for explaining a method of detecting the branch point 4.

The branch point 4 is detected by a change in the number of blood vessels detected in the measurement range of the ultrasonic probe 16. In A-mode data 40 of an ultrasonic transducer row 20 to measure the vascular short-axis cross section, relatively high signal strength is obtained in a low depth region and a vessel wall, and an intravascular lumen equivalent portion 44 having extremely low signal strength that is specific to the intravascular lumen is present between the peaks of the signal strength that are generated by the reflected wave due to the vessel wall. In the present embodiment, for each measurement rate, 1) peaks at which the signal strength is equal to or greater than a predetermined reference value are extracted for each ultrasonic transducer, 2) a pair is made using the extracted peaks, 3) the number of pairs of peaks having an appropriate gap as the carotid artery 3 and having the intravascular lumen equivalent portion 44, in which the average value of the signal strength between the peaks of the pair is equal to or less than a reference value Plow, is counted as the number of blood vessels, and 4) it is determined that the branch point 4 has been detected when the counted number becomes 1 from 2. The branch point 4 is detected while moving the ultrasonic transducer row 20 from the position of the thick dashed line on the upper side to the position of the thick dashed line on the lower side in Fig. 3. A specific example of the A-mode data 40 at the position of the thick dashed line on the lower side is shown in Fig. 3. Here, the number of peak pairs is "1". Accordingly, the intravascular lumen equivalent portion 44 is "1". That is, the number of blood vessels is "1". In addition, the intravascular lumen equivalent portion 44 may be detected instead of detecting the pair of peaks.

When the position of the ultrasonic transducer row 20 is a position shown in Fig. 3, the internal carotid artery 3a and the external carotid artery 3b are included in the measurement range of the ultrasonic transducer row 20. When viewed from the ultrasonic probe 16, the number of intravascular lumen equivalent portions 44 that may be detected regardless of whether the internal carotid artery 3a and the external carotid artery 3b overlap each other or are located side by side in the depth direction, that is, the number of pairs of peaks (the number of detected blood vessels) is "2". As indicated by a thick black arrow, when the ultrasonic probe 16 is moved in the torso direction of the subject 2 and the ultrasonic transducer row 20 finally reaches the branch point 4 of the carotid artery, the number of intravascular lumen equivalent portions 44, that is, the number of pairs of peaks (the number of detected blood vessels) becomes "1", and accordingly, the branch point 4 is detected.

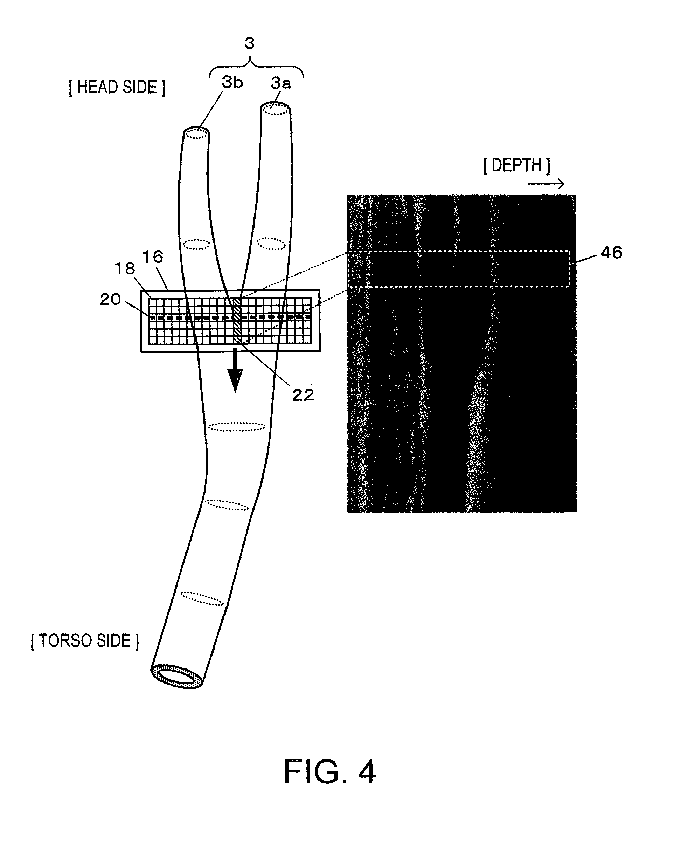

Fig. 4 is a diagram for explaining the calculation of the moving distance of the ultrasonic probe 16 along the blood vessel from the detection timing of the branch point 4.

When the branch point 4 is detected, the ultrasonic measurement apparatus 10 extracts an ultrasonic transducer having the longest intravascular lumen equivalent portion 44, as a transducer immediately above a blood vessel, from the measurement data of each transducer of the ultrasonic transducer row 20. Then, ultrasonic measurement is started with ultrasonic transducers in an ultrasonic transducer column 22 that crosses the ultrasonic transducer row 20 so as to include the transducer immediately above a blood vessel, and a B-mode image 46 is generated for each measurement frame. The size (length in a direction along the skin surface) of the B-mode image 46 generated by one frame depends on the number of ultrasonic transducers in the short side direction among the ultrasonic transducers arrayed in a two-dimensional manner. In the example shown in Fig. 4, this is a region of a white dashed rectangle.

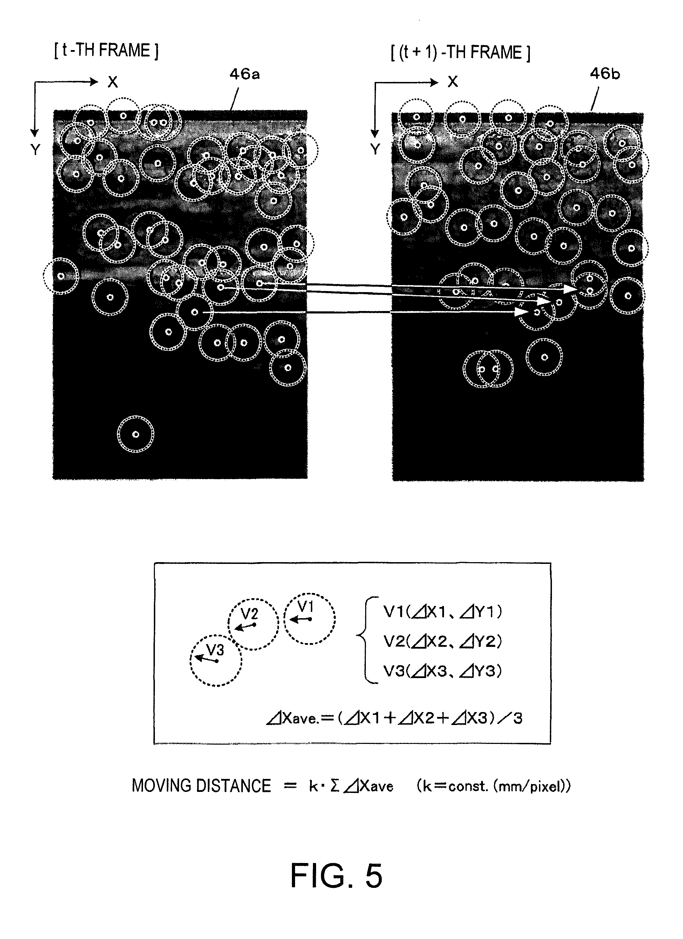

Then, as shown in Fig. 5, the ultrasonic measurement apparatus 10 extracts a feature point (center position of the dashed circle in Fig. 5) in each of the newly generated B-mode image 46b and the B-mode image 46a generated by the last frame, and performs feature point matching estimation between the two B-mode images. Fig. 5 shows the correspondence relationship estimated by the arrow. In the example shown in Fig. 5, matching of three points is performed. Since the feature point extraction method and the matching estimation method can be realized by using known techniques appropriately, explanation thereof herein will be omitted.

Then, if the matching of feature points is possible, the ultrasonic measurement apparatus 10 calculates displacement vectors V1, V2, and V3 between two B-mode images for each matched feature point, and calculates the average amount of displacement ΔXave in a vascular longitudinal direction (X-axis direction in the B-mode image coordinate system). The average amount of displacement ΔXave is calculated for each frame and is integrated after the branch point 4 is detected. Then, the integrated value of displacement ΣΔXave is multiplied by a known constant k for conversion into the actual distance (millimeters) corresponding to the number of pixels of the B-mode image, and the result is set as a moving distance of the ultrasonic probe 16 after the branch point 4 is detected. Then, at a place where the moving distance has amounted to the predetermined distance L (refer to Fig. 2), the ultrasonic measurement apparatus 10 performs predetermined notification, and starts the measurement of the vascular function information.

### Description of functional configuration

Next, the functional configuration for realizing the present embodiment will be described.

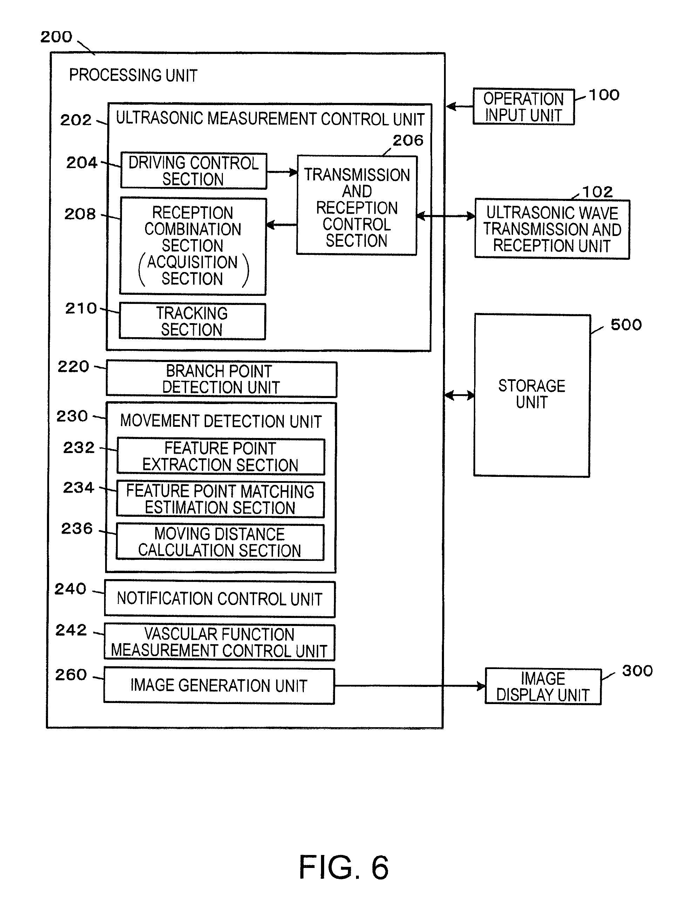

Fig. 6 is a block diagram showing an example of the functional configuration of the ultrasonic measurement apparatus 10 in the present embodiment. The ultrasonic measurement apparatus 10 includes an operation input unit 100, an ultrasonic wave transmission and reception unit 102, a processing unit 200, an image display unit 300, and a storage unit 500.

The operation input unit 100 receives various kinds of operation input by the operator, and outputs an operation input signal corresponding to the operation input to the processing unit 200. The operation input unit 100 can be implemented by a button switch, a lever switch, a dial switch, a track pad, a mouse, or the like. In the example shown in Fig. 1, the touch panel 12 or the keyboard 14 corresponds to the operation input unit 100.

The ultrasonic wave transmission and reception unit 102 transmits an ultrasonic wave with a pulse voltage output from the processing unit 200. Then, the ultrasonic wave transmission and reception unit 102 receives a reflected wave of the transmitted ultrasonic wave, converts the reflected wave into a reflected wave signal, and outputs the reflected wave signal to the processing unit 200. The ultrasonic probe 16 shown in Fig. 1 corresponds to the ultrasonic wave transmission and reception unit 102.

The processing unit 200 is realized by a microprocessor, such as a CPU or a GPU, or an electronic component, such as an ASIC or an IC memory, for example. In addition, the processing unit 200 performs control of the input and output of data to each functional unit, and calculates biological information of the subject 2 by performing various kinds of arithmetic processing based on a predetermined program or data, the operation input signal from the operation input unit 100, the reflected wave signal from the ultrasonic wave transmission and reception unit 102, or the like. The processing device 30 and the control board 31 shown in Fig. 1 correspond to the processing unit 200.

In the present embodiment, the processing unit 200 includes an ultrasonic measurement control unit 202, a branch point detection unit (detection unit) 220, a movement detection unit 230, a notification control unit (notification unit) 240, a vascular function measurement control unit 242, and an image generation unit 260.

The ultrasonic measurement control unit 202 controls the transmission of an ultrasonic wave toward the blood vessel and the reception of a reflected wave. For example, the ultrasonic measurement control unit 202 includes a driving control section 204, a transmission and reception control section 206, a reception combination section (acquisition unit or acquisition section) 208, and a tracking section 210, and performs overall control of ultrasonic measurement. The ultrasonic measurement control unit 202 can be realized by known techniques.

The driving control section 204 controls the transmission timing of ultrasonic pulses from the ultrasonic probe 16, and outputs a transmission control signal to the transmission and reception control section 206.

The transmission and reception control section 206 generates a pulse voltage according to the transmission control signal from the driving control section 204, and outputs the pulse voltage to the ultrasonic wave transmission and reception unit 102. In this case, it is possible to adjust the output timing of the pulse voltage to each ultrasonic transducer by performing transmission delay processing. In addition, it is possible to perform amplification or filtering of the reflected wave signal output from the ultrasonic wave transmission and reception unit 102 and to output the result to the reception combination section 208.

The reception combination section 208 performs processing relevant to the so-called focus of a received signal by performing delay processing as necessary, thereby generating reflected wave data.

The tracking section 210 performs processing relevant to so-called "tracking" that is for tracking the position of a region of interest between frames of ultrasonic measurement based on the reflected wave data (reflected wave signal). For example, it is possible to perform processing for setting a region of interest (tracking point) in the reflected wave data (for example, a B-mode image) as a reference, processing for tracking each region of interest between different frames, and processing for calculating the displacement for each region of interest. Functions, such as so-called "echo tracking" or "phase difference tracking" that is known, are realized.

The branch point detection unit 220 performs control relevant to the detection process for detecting a blood vessel part (in the present embodiment, the branch point 4) satisfying the predetermined tissue conditions.

The movement detection unit 230 performs control relevant to the distance measuring process for detecting that the ultrasonic wave transmission and reception unit 102 has moved on the skin surface along the longitudinal direction of the blood vessel and has reached a predetermined position for performing predetermined vascular function measurement, from the detection of the branch point detection unit 220.

Specifically, the movement detection unit 230 includes a feature point extraction section 232 that extracts a feature point from the B-mode image for each measurement frame, a feature point matching estimation section 234 that performs feature point matching between frames, and a moving distance calculation section 236 that calculates and integrates a moving distance between the frames of matched feature points.

The notification control unit 240 performs control for notifying that the detection has been made by the movement detection unit 230. In the present embodiment, a predetermined information image is displayed on the image display unit 300.

The vascular function measurement control unit 242 performs control relevant to the measurement of predetermined vascular function information and control to store the measurement result in the storage unit 500 and display the measurement result on the image display unit 300.

The image generation unit 260 generates an image for displaying a measurement result or various operation screens required for ultrasonic measurement or biological information measurement, and outputs the image to the image display unit 300.

The image display unit 300 displays image data input from the image generation unit 260. The touch panel 12 shown in Fig. 1 corresponds to the image display unit 300.

The storage unit 500 is realized by a storage medium, such as an IC memory, a hard disk, or an optical disc, and stores various programs or various kinds of data, such as data in the operation process of the processing unit 200. In Fig. 1, the storage medium 33 mounted in the control board 31 of the processing device 30 corresponds to the storage unit 500. In addition, the connection between the processing unit 200 and the storage unit 500 is not limited to a connection using an internal bus circuit in the apparatus, and may be realized by using a communication line, such as a local area network (LAN) or the Internet. In this case, the storage unit 500 may be realized by using an external storage device separate from the ultrasonic measurement apparatus 10.

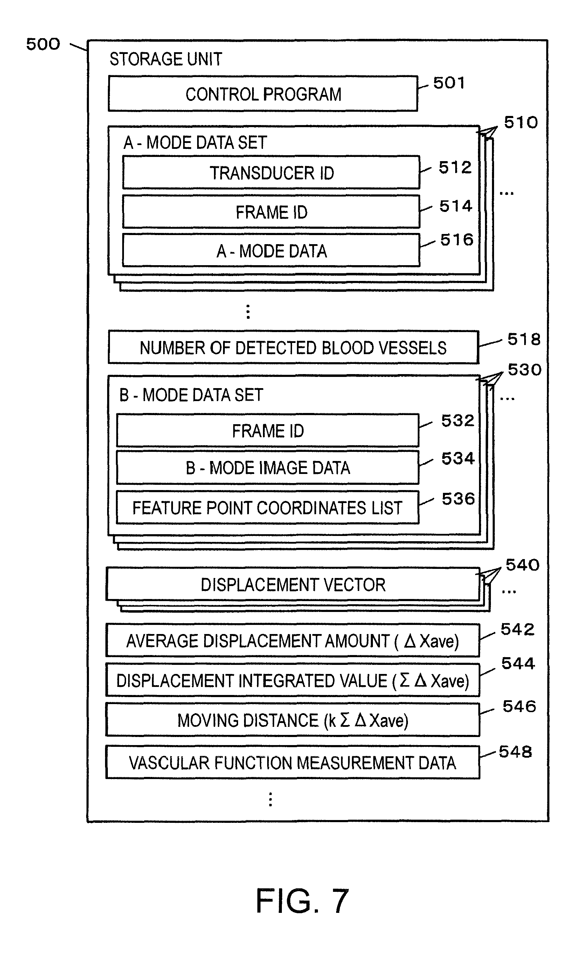

In addition, as shown in Fig. 7, the storage unit 500 stores a control program 501, an A-mode data set 510, the number of detected blood vessels 518, a B-mode data set 530, a displacement vector 540, an average displacement amount 542, a displacement integrated value 544, a moving distance 546, and vascular function measurement data 548. Needless to say, frame identification information, various flags, counter values for time checking, and the like other than those described above can also be appropriately stored.

The control program 501 causes the processing unit 200 to realize the functions of the ultrasonic measurement control unit 202, the branch point detection unit 220, the movement detection unit 230, the notification control unit 240, the vascular function measurement control unit 242, the image generation unit 260, and the like. In addition, when realizing these functional units with hardware, such as an electronic circuit, a part of the program for realizing the function can be omitted.

The A-mode data set 510 is generated for each ultrasonic transducer in the ultrasonic transducer row 20 (refer to Fig. 3) that is used for the measurement in the branch point detection process. One A-mode data set 510 includes a transducer ID 512, a frame ID 514, and A-mode data 516 (depth-signal strength data). Needless to say, data other than these can also be appropriately stored.

The number of detected blood vessels 518 is the number of blood vessels present in the measurement range of the ultrasonic transducer row 20 (refer to Fig. 3) in the branch point detection process.

The B-mode data set 530 is generated for each ultrasonic transducer in the ultrasonic transducer column 22 (refer to Fig. 4) that is used for the measurement in the distance measurement process. One B-mode data set 530 includes a frame ID 532, B-mode image data 534, and a feature point coordinates list 536. Needless to say, data other than these can also be appropriately stored.

### Description of the flow of the process

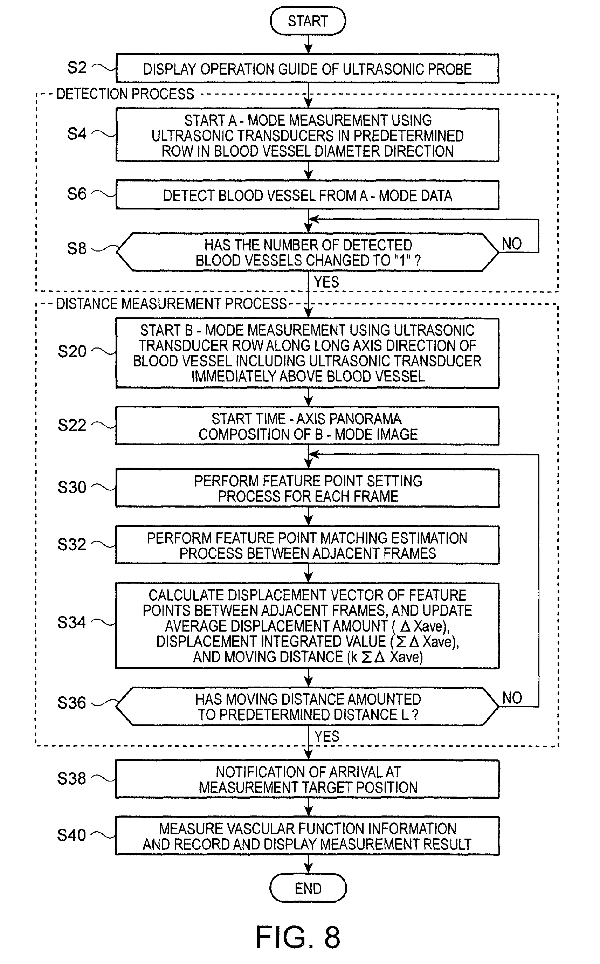

Fig. 8 is a flowchart for explaining the operation up to vascular function measurement. This is realized by the execution of the control program 501 by the processing unit 200.

First, the processing unit 200 displays the predetermined operation guide of the ultrasonic probe 16 on the touch panel 12 (step S2). That is, the operator is prompted to perform an operation of placing the ultrasonic probe 16 at a position on the carotid artery 3 near the head in a relative posture, in which the vascular longitudinal direction and the longitudinal direction of the ultrasonic wave transmitting and receiving surface 18 of the ultrasonic probe 16 are perpendicular to each other, and of sliding the ultrasonic probe 16 on the skin face slowly in the torso direction along the carotid artery 3 while maintaining the posture.

Then, the processing unit 200 performs a step relevant to the process of detecting the branch point 4.

That is, A-mode measurement using the ultrasonic transducer row 20 of a predetermined long side direction row is started (step S4). Then, a blood vessel portion (a pair of peaks or the intravascular lumen equivalent portion 44) is detected by analyzing the A-mode data 516, and the number of blood vessel portions is stored in the number of detected blood vessels 518 (step S6).

If the number of detected blood vessels 518 changes to "1" (YES in step S10), the processing unit 200 determines that the ultrasonic probe has reached the branch point 4, and starts processing relevant to the distance measurement process.

That is, B-mode measurement using the ultrasonic transducer column 22 (refer to Fig. 4) along the longitudinal direction of the blood vessel including the transducer immediately above a blood vessel is started (step S20), and the generation of a time-axis panorama composite image of the obtained B-mode image is started (step S22). Then, a feature point setting process is performed for each frame (step S30), and feature point matching estimation between frames is performed (step S32).

Then, the displacement vector 540 (refer to Figs. 5 and 7) between frames is calculated for each matched feature point, and the average displacement amount 542, the displacement integrated value 544, and the moving distance 546 are sequentially updated (step S34).

Then, when it is detected that the moving distance 546 has amounted to the predetermined distance L (YES in step S36), the processing unit 200 perform predetermined notification control (step S38: notification process). The operator becomes aware that the ultrasonic probe 16 has reached the measurement target position by the notification, and stops the movement of the ultrasonic probe 16.

When an operation input for starting the measurement of predetermined vascular function information is detected (or when it is measured that a predetermined amount of time has passed from the notification information), the processing unit 200 starts the measurement of the vascular function information with the ultrasonic probe 16 and performs recording and display of the measurement result (step S40).

As described above, according to the present embodiment, it is possible to easily realize steady and accurate positioning for the blood vessel to be subjected to ultrasonic measurement.

### Modification examples

In addition, embodiments of the invention are not limited to the embodiment described above, and constituent components can be appropriately added, omitted, and changed.

For example, although the ultrasonic transducers of the ultrasonic probe 16 are arrayed in a two-dimensional manner in the embodiment described above, the arrangement of the ultrasonic transducers of the ultrasonic probe 16 is not limited thereto. For example, it is also possible to adopt an H-type arrangement in which three rows of ultrasonic transducers are combined or a T-type arrangement in which two rows of ultrasonic transducers are combined. For example, in the case of the H-type arrangement, it is preferable to use one of the two vertical rows in the H-type arrangement for detection of the branch point 4 and to use one horizontal row in the distance measurement process. The opposite is also possible. In the case of the T-type arrangement, it is preferable to use a certain one row for detection of the branch point 4 and to use the other row in the distance measurement process.

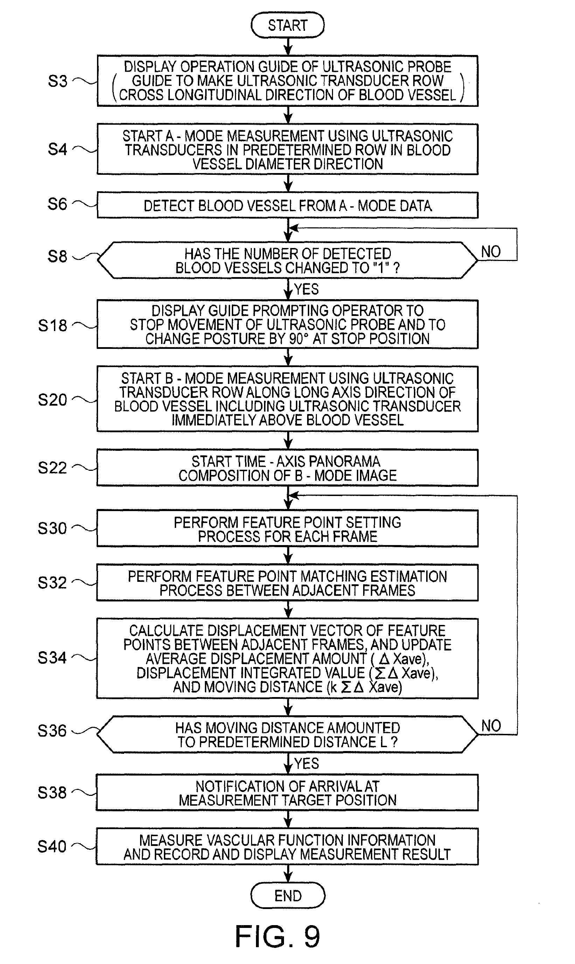

In addition, ultrasonic transducers may be arrayed in one row. In this case, as shown in Fig. 9, instead of step S2, an operation guide prompting the operator to make the ultrasonic transducer row cross (preferably, be perpendicular to) the longitudinal direction of the blood vessel is displayed (step S3).

Before step S20, the movement of the ultrasonic probe 16 is stopped, and the posture is changed so that the direction of the ultrasonic probe 16 is changed by 90° at the location. Accordingly, the row of ultrasonic transducers is arrayed in a direction along the longitudinal direction of the carotid artery 3. Then, a guide prompting the operator to move the ultrasonic probe 16 so as to be slowly moved in the torso direction of the subject 2 while maintaining the posture is displayed (step S18). Then, it is preferable to perform the process from step S20 after the posture of the ultrasonic probe 16 is actually changed and a predetermined operation input indicating that the posture change has been completed is made.

In the embodiment described above, the carotid artery has been mentioned as an example of the measurement target. However, the embodiment can apply to, in addition to the carotid artery, other blood vessels having a branch point (satisfying tissue conditions), such as the subclavian artery, as measurement targets.

The entire disclosure of Japanese Patent Application No. 2014-031418, filed on February 21, 2014 is expressly incorporated by reference herein.

## Claims

1. An ultrasonic measurement apparatus (10), comprising:
an ultrasonic wave transmission and reception unit (102) that transmits and receives an ultrasonic wave to and from a blood vessel;
an acquisition unit (208) that acquires measurement data, which is obtained by measuring the blood vessel with an ultrasonic wave, based on a received signal of the ultrasonic wave transmission and reception unit (102);
a detection unit (220) that detects the blood vessel based on the measurement data; and
a movement detection unit (230) that detects an arrival at a predetermined position by movement along the blood vessel based on the measurement data, the predetermined position being a position for performing vascular function measurement of the blood vessel.

2. The ultrasonic measurement apparatus (10) according to claim 1, wherein the detection unit (220) detects a branch point (4) at which the blood vessel branches into branch blood vessels.

3. The ultrasonic measurement apparatus (10) according to claim 1 or 2, wherein the movement detection unit (230) calculates a moving distance by comparing the measurement data in time series, and detects an arrival at the predetermined position when the moving distance from detection of the detection unit (220) has amounted to a predetermined distance.

4. The ultrasonic measurement apparatus (10) according to any one of claims 1 to 3, wherein
the detection unit (220) detects the blood vessel based on the measurement data relevant to a cross section of the blood vessel in a short-axis direction of the blood vessel, and
the movement detection unit (230) detects an arrival at the predetermined position based on the measurement data relevant to a cross section of the blood vessel in a long-axis direction of the blood vessel.

5. The ultrasonic measurement apparatus (10) according to any one of the preceding claims, further comprising a notification unit (240) that notifies that detection has been made by the movement detection unit (230).

6. The ultrasonic measurement apparatus (10) according to any one of the preceding claims, wherein
the blood vessel is a carotid artery (3), and
the detection unit (220) detects a branch point (4) of an internal carotid artery (3a) and an external carotid artery (3b).

7. An ultrasonic measurement method, comprising:
acquiring measurement data, which is obtained by measuring a blood vessel with an ultrasonic wave, based on a received signal of an ultrasonic wave transmission and reception unit (102) for transmitting and receiving an ultrasonic wave to and from the blood vessel;
detecting the blood vessel based on the measurement data; and
detecting an arrival at a predetermined position by movement of the ultrasonic wave transmission and reception unit (102) along the blood vessel based on the measurement data, the predetermined position being a position for performing vascular function measurement of the blood vessel.

8. The ultrasonic measurement method according to claim 7, wherein the detecting of the blood vessel includes the detecting of a branch point at which the blood vessel branches into branch blood vessels.

9. The ultrasonic measurement method according to claim 7 or 8, wherein a moving distance is calculated by comparing the measurement data in time series, and the arrival at the predetermined position is detected when the moving distance from detection of the detection unit has amounted to a predetermined distance.

10. The ultrasonic measurement method according to any one of claims 7 to 9, wherein
the blood vessel is detected based on the measurement data relevant to a cross section of the blood vessel in a short-axis direction of the blood vessel, and
the arrival at the predetermined position is detected based on the measurement data relevant to a cross section of the blood vessel in a long-axis direction of the blood vessel.

11. The ultrasonic measurement method according to any one of claims 7 to 10, wherein an operator is notified of an arrival at the predetermined position

12. The ultrasonic measurement method according to any one of claims 7 to 11, wherein
the blood vessel is a carotid artery (3), and
the detecting of the blood vessel includes the detecting of a branch point (4) of an internal carotid artery (3a) and an external carotid artery (3b).
